# EUROPÄISCHE PATENTSCHRIFT

(11) **EP 0 793 480 B1**
(45) Veröffentlichungstag und Bekanntmachung des Hinweises auf die Patenterteilung: **14.10.1998**
(21) Anmeldenummer: 95940119.1
(22) Anmeldetag: 23.11.1995
(51) Int. Cl.: A61K 7/42, A61K 7/48

(54) **SONNENSCHUTZZUBEREITUNG MIT ERHÖHTEM LICHTSCHUTZFAKTOR**
SUN-PROTECTION PREPARATION WITH INCREASED LIGHT-PROTECTION FACTOR
COMPOSITION DE PROTECTION SOLAIRE A INDICE DE PROTECTION RENFORCE

(30) Priorität: 24.11.1994 DE 4443243
(43) Veröffentlichungstag der Anmeldung: 10.09.1997
(73) Patentinhaber: LANCASTER GROUP GmbH, 67059 Ludwigshafen (DE)
(72) Erfinder: STANZL, Klaus, White Plains, NY 10605 (US); ZASTROW, Leonhard, MC-98000 Monaco (MC); KULKARNI, Rupali, Bridgewater, NJ 08807 (US); CERNASOV, Domnica, Ringwood, NJ 07456 (US)
(74) Vertreter: Walter, Wolf-Jürgen
(86) Internationale Anmeldenummer: DE9501701
(87) Internationale Veröffentlichungsnummer: WO9615772

(56) Entgegenhaltungen:
- EP-A- 0 197 485
- EP-A- 0 430 473
- WO-A-93/11095
- WO-A-93/11742
- WO-A-94/18940

## Beschreibung

Die Erfindung betrifft eine Sonnenschutzzubereitung mit erhöhtem Lichtschutzfaktor.

Es sind bereits eine Vielzahl von Sonnenschutzzubereitungen bekannt geworden. Im allgemeinen fallen topische Sonnenschutzformulierungen in eine der Kategorien chemische oder physikalische Sonnenschutzformulierungen. Die hier interessierenden chemischen Sonnenschutzformulierungen enthalten eine oder mehrere UV-absorbierende Stoffen und wirken nach Aufbringen eines dünnen und unsichtbaren Filmes auf der Haut als Filter, wobei sie den Durchgang von ultravioletter Strahlung zu den lebenden Zellen der Epidermis nicht gestatten. Chemische Sonnenschutzformulierungen liegen Üblicherweise farblos vor, da sie keine Stoffe enthalten, die das sichtbare Licht absorbieren, und sie sind daher für die meisten Personen kosmetisch annehmbar, vorausgesetzt daß sie Haut und Augen nicht reizen, nicht fotosensibilisierend sowie stabil und nicht flüchtig sind und keine Flecken auf Haut und Bekleidung hinterlassen. Die meisten der kommerziellen Sonnenschutzformulierungen enthalten eine oder mehrere Chemikalien, die die Ultraviolett B-Strahlung absorbieren, und sie enthalten weiterhin eine feuchtigkeitsvermittelnde Grundlage. In jüngster Zeit enthalten bekannte Sonnenschutzformulierungen auch Stoffe, die Ultraviolett A-Strahlung absorbieren, insbesondere sind dies verschiedene Benzophenone. Die am meisten eingesetzten chemischen Sonnenschutzformulierungen enthalten p-Aminobenzoesäure (PABA), PABA-Ester (Amyldimethyl-PABA und Octyldimethyl-PABA), Benzophenone, Cinnamate (Octylmethoxycinnamat und -cinnoxat), Salicylate (Homomenthylsalicylat) und Anthranilate.

Aus der WO 94/06409 (PCT/US92/11037) für Perricone et al. sind Sonnenschutzformulierungen bekannt, die α-Hydroxysäuren oder deren Derivate enthalten. Weiterhin sind aus der WO93/19729 (PCT/US93/02712) Sonnenschutzformulierungen bekannt, die Retinoinsäure sowie verschiedene Derivate davon zusammen mit Arzneimitteln enthalten.

Ein Vorschlag zum Einsatz von Titandioxid und/oder Zinkoxid mit einem Anteil von 0,5 bis 30 Gewichts-% sowie Eisenoxid mit einem Anteil von 0,001 bis 2 Gewichts-%, wobei die mittlere primäre Teilchengröße des Eisenoxids etwa 200 nm beträgt, wurde in der WO93/11742 (PCT/EP92/02897) gemacht. Diese Teilchen können gegebenenfalls mit Phospholipiden überzogen sein.

Bei den Sonnenschutzformulierungen hat sich im allgemeinen die Angabe des Lichtschutzfaktors durchgesetzt. Es besteht ein großes Interesse, mit möglichst einfachen Maßnahmen den Lichtschutzfaktor soweit zu erhöhen, daß das Produkt bei einem gleichbleibend guten Hautgefühl für einen großen Personenkreis verwendbar ist. Besonders interessant sind dabei Lichtschutzfaktoren im mittleren Bereich von LSF 10 bis 20.

Der Erfindung liegt daher die Aufgabe zugrunde, neue und hinsichtlich des Lichtschutzfaktors verbesserte Sonnenschutzzubereitungen zur Verfügung zu stellen.

Erfindungsgemäß besteht eine Sonnenschutzzubereitung mit erhöhtem Lichtschutzfaktor aus
(a) einem feinteiligen, vernetzten Poly(Methylsiloxan) mit einer Teilchengröße im Bereich von 1 bis 10 µm;
(B) Octyldodecylneopentanoat;
(C) Formulierungshilfen; und
(4) Additiven;
wobei das Verhältnis von (A) zu (B) im Bereich von 1 : 2 bis 10 liegt, und der Anteil von (A) und (B) im Bereich von 3 bis 12 Gewichts-% liegt, bezogen auf die Gesamtzusammensetzung.

Eine besonders vorteilhafte Zusammensetzung enthält die Komponente (A) mit einer mittleren Teilchengröße von 4,5 µm.

Bei einer weiteren bevorzugten Ausführungsform beträgt das Verhältnis (A) zu (B) 1 : 2,5 bis 8, vorzugsweise 1 : 3 bis 5.

Eine weitere bevorzugte Ausführungsform enthält die Komponenten (A) und (B) in einem Gesamtanteil von 4 bis 8 Gewichts%, bezogen auf die Gesamtzusammensetzung.

Bei dem feinteiligen, vernetzten Poly(Methylsiloxan) als Komponente (A) handelt es sich um ein Produkt, das insbesondere folgende Eigenschaften hat:
- Gewichtsverlust bei 350 °C von 3% oder weniger und bei 900 °C von 12% oder weniger;
- keine Veränderung des Teilchendurchmessers bei Calcinierung von Luft über vier Stunden bis etwa 350 °C, Verringerung des Teilchendurchmessers bis 900 °C um etwa 25% oder weniger, jeweils unter Beibehaltung der im wesentlichen runden Struktur;
- Fehlen eines endothermen Peaks beim Aufheizen in Luft mit einer Heizgeschwindigkeit von 10 °C/Minute;
- Beständigkeit gegen organische Lösungsmittel auf Basis von Alkoholen, Ketonen, Estern, Aromaten und chlorierten Kohlenwasserstoffen.

Ein besonders vorteilhaftes Poly(Methylsiloxan) ist das Handelsprodukt Tospearl, insbesondere Tospearl 145 (von Toshiba Silicone Co., Ltd., erhältlich von Kobo Products, Inc., NJ, USA).

Von der Komponente (B) Octyldodecylneopentanoat (CTFA-Name) ist bekannt, daß sie ein Emollient ist. Ein besonders vorteilhaftes Handelsprodukt ist unter dem Namen Elefac I 205 (erhältlich von Bermel Chem. Comp., Englewood, N.J., USA) verfügbar. Man erhält beispielsweise bei Einsatz dieser Komponente (B) in einer Formulierung mit UV-Filtern und anderen üblichen Hilfsstoffen und Additiven einen Lichtschutzfaktor von etwa 16.

Überraschenderweise wird der Lichtschutzfaktor durch Zusatz geringer Mengen des Poly(Methylsiloxan) als Komponente (A) auf ca. 18 bis 19 erhöht, was nicht zu erwarten war. Es ist zwar bekannt, daß das vernetzte Polymere (A) eine hohe Wärmebeständigkeit hat, jedoch gibt es keine Aussage zum Einfluß auf UV-Strahlung.

Die Komponenten (A) und (B) können in übliche Liposomen verkapselt werden.

Eine besonders vorteilhafte Ausführungsform besteht darin, daß die Komponenten (A) und (B) in asymmetrischen lamellaren Aggregaten vorliegen, die aus Phospholipiden und mit Sauerstoff beladenen Fluorcarbonen oder Fluorcarbongemischen bestehen, wobei der Anteil an Fluorcarbon im Bereich von 0,2 bis 100% w/v liegt und die einen Phosphatidylcholingehalt der Lipidfraktion von 30 bis 99 Gewichts-% haben und die weiterhin eine Hautpenetrierung in Abhängigkeit von der kritischen Löslichkeitstemperatur (in n-Hexan) der Fluorcarbone aufweisen. Die Einbeziehung der Komponenten (A) und (B) in die genannten neuartigen asymmetrischen lamellaren Aggregate, die aus der WO94/00098 bekannt sind, kann ein besonders tiefes Eindringen der für die UV-Strahlung entscheidenden Komponenten gestatten, und dadurch zusätzlich zu einer weiteren Verbesserung des Lichtschutzes beitragen.

Im Unterschied zu den bekannten wäßrigen Liposomen (Vesikel) tragen diese Phospholipidstabilisierten Aggregate in ihrem Kern hydrophobe Fluorcarbone, die zum Transport von Sauerstoff befähigt sind. Ihre grenzflächenchemische Stabilisierung erfolgt primär durch eine Monolayer mit inverser Anordnung und gegebenenfalls ein sich daran anschließender Aufbau von Bilayer-Schichten. Wegen der Besonderheit ihrer strukturellen Anordnung werden diese neuartigen Aggregate als asymmetrische lamellare Sauerstoff-Carrier bezeichnet. Ihre außergewöhnliche kolloidchemische Stabilität ist vermutlich auf die lamellare Struktur und auf die Oberflächenladung der Aggregate zurückzuführen. Letztere ist auf die Auswahl geeigneter Phospholipide beziehungsweise deren Mischungen natürlicher wie auch synthetischer Provenienz zurückzuführen. In erster Linie sind für eine vorteilhafte Wirkung in diesem Sinne Phospholipide, insbesondere Phosphatidylcholin im genannten Konzentrationsbereich von 30 bis 99 % in Verbindung mit Lysolecithinen der Konzentration von 0,1 bis 10 % und/oder geladenen Phospholipiden im Konzentrationsbereich 0,1 bis 30 Gew.-% verantwortlich. Die angesprochene Wirkung der Phospholipide wird durch entsprechende negative Zeta-Potentiale und durch die Messung von Ladungsdichten (bei Titration mit einem kationischen Polyelektrolyten) verifiziert. Wesentlich für den Einsatz-der Fluorcarbon-Aggregate ist die Hautpenetrierung in Abhängigkeit von der kritischen Löslichkeitstemperatur der aus gewählten Fluorcarbone oder Fluorcarbongemische (für den Einsatz asymmetrischer lamellarer Aggregate siehe auch DE-B-42 21 255).

Der Anteil der mit den lichtschützenden Bestandteilen beladenen Aggregate kann im Bereich von 5 bis 60 Gew.-% liegen, bezogen auf die Gesamtzubereitung, und liegt vorteilhaft im Bereich von 10 bis 50 Gew.-%, insbesondere 30 bis 50 Gew.%.

Wie bereits ausgeführt, können auch übliche Liposome als Transportsystem für das modifizierte kaolinhaltige Gemisch innerhalb der erfindungsgemäßen Zubereitung eingesetzt werden. Liposome sind vollständig geschlossene Lipid-Bilayer-Membranen, die ein wäßriges Volumen eingeschlossen enthalten. Liposome können unilamellare Vesikel sein (die eine Einzelmembran-Bilayer besitzen) oder multilamellare Vesikel (Onion-ähnliche Strukturen, gekennzeichnet durch Mehrfachmembran-Bilayer, von denen jede von der nächsten durch eine wäßrige Schicht getrennt ist). Die Bilayer besteht aus zwei Lipid-Monolayern, die einen hydrophoben "Schwanz"-Bereich und einen hydrophilen "Kopf"-Bereich haben. Die Struktur der Membran-Bilayer ist so, daß die hydrophoben (unpolaren) "Schwänze" der Lipidmonolayer sich in Richtung des Zentrums der Bilayer orientieren, während sich die hydrophilen "Köpfe" in Richtung der wäßrigen Phase orientieren.

Die Herstellung von Liposomen, aus gesättigten und ungesättigten Lipiden, ist in sehr vielen Patenten beschrieben worden, ebenso deren Einsatz als Transportsystem. Die Einarbeitung des modifizierten kaolinhaltigen Gemisches kann auf übliche Weise erfolgen.

Als weitere Bestandteile der erfindungsgemäßen Sonnenschutzzubereitung sind zusätzliche UVB-Filter zu nennen, die öllöslich oder wasserlöslich sein können. Dazu gehören beispielsweise 4-Aminobenzoesäure-Derivate, Ester der Zimtsäure, Ester der Salicylsäure, Derivate des Benzophenons sowie Sulfonsäurederivate des Benzophenons, Sulfonsäurederivate, Sulfonsäurederivate des 3-Benzylidencamphers usw. Weiterhin können anorganische Pigmente eingesetzt werden, wie Oxide von Titan, Zink, Eisen, Zirkonium, Silicium, Mangan, Aluminium und Mischungen davon. Bei der Einbeziehung anorganischer Pigmente ist allerdings zu beachten, daß deren Agglomeration durch erhöhten Zusatz an Emulgatoren auszugleichen ist. Es können auch bekannte Radikalfänger, wie α-Tocopherol oder Tocopherylacetat eingesetzt werden. Da es sich bei den fotochemischen Reaktionsprodukten meist um Radikale von Verbindungen handelt, z.B. Hydroxyradikale, Hydroxyperoxyradikale oder Superoxid-Ionen sowie Singulettsauerstoff, können auch weitere Radikalfänger, die für kosmetische Zubereitungen geeignet sind, wie zum Beispiel trans-Urocaninsäure verwendet werden.

Die erfindungsgemäße Zubereitung enthält darüber hinaus weitere Hilfsmittel, die für ähnliche Zubereitungen üblich sind. Dazu gehören Stabilisatoren, wie PEG-8, BHT (Protegol) vernetzte Alkylacrylate (Pemulen) sowie Konservierungsmittel, wie Phenoxyethanol, Parabene, Methyldibromglutaronitril, Benzophenon-3 usw., weitere Gele und Parfümöle. Zusätzlich können auch Extrakte von Pflanzen, wie Aloe vera und anderen enthalten sein.

Die Herstellung der erfindungsgemäßen Sonnenschutzzubereitung mit erhöhtem Lichtschutzfaktor erfolgt durch ein Emulgierungsverfahren, bei dem die Bestandteile, gegebenenfalls nach vorheriger separater Emulgierung z.B. bei erhöhter Temperatur anschließend zusammen vermischt werden. Dabei ist es vorteilhaft, die Komponente (A) unmittelbar nach der Öl-Phase während des Emulgierungsverfahrens hinzuzugeben. Nach ausreichender Homogenisierung wird das Homogenisat auf Raumtemperatur abgekühlt, und es werden die Konservierungsmittel und Parfümöle hinzugegeben. Eine keimfreie Verarbeitung ist selbstverständlich zu gewährleisten.

Für den Fall, wo die erfindungsgemäße Sonnenschutzzubereitung die oben genannten asymmetrischen lamellaren Aggregate enthält, werden die Komponenten (A) und (B) nacheinander in ein Perfluorcarbon, gegebenenfalls vermischt mit Glycerin und Propylenglycol, unter guter Homogenisierung eingetragen, und es wird ein Phospholipid mit einem Phosphatidylcholingehalt von mehr als 30% in dieses Homogenisat eingerührt und anschließend Wasser hinzugegeben. Nach ausreichend guter Homogenisierung stehen die mit den lichtschützenden Bestandteilen beladenen Aggregate für die weitere Einarbeitung in die Suspension zur Verfügung. Sofern weitere Lichtschutzbestandteile in der fertigen Zubereitung enthalten sein sollen, können diese ebenfalls in diese Aggregate eingearbeitet werden.

Eine besonders vorteilhafte Anwendungsform der erfindungsgemäßen Sonnenschutzzubereitung kann als Emulsion oder als Spray vorliegen. Für den Fall der Formulierung als Spray können der erfindungsgemäßen Zubereitung noch übliche, für einen Spray bekannte Stoffe wie Treibmittel, insbesondere umweltfreundliche Treibmittel, zugesetzt werden, so daß das fertige Produkt in einem für die Anwendung fertigen Aerosolbehälter vorliegt.

Die Erfindung soll nachstehend durch Beispiele näher erläutert werden. Die in den Beispielen genannten Prozentangaben sind auf das Gewicht bezogen.

### Beispiel 1

| | |
|---|---|
| Phenyltrimethicon | 5,00 % |
| Tocopherylacetat | 2,00 % |
| Octyldodecylneopentanoat | 4,00 % |
| Bisabolol | 0,50 % |
| Isostearylneopentanoat | 1,50 % |
| BHT | 0,07 % |
| Pemulen TR 2 | 0,25 % |
| Benzophenon-3 | 2,00 % |
| Octylmethoxycinnamat | 7,50 % |
| 2-Phenylbenzimidazol-5-sulfonsäure | 16,00 % |
| Panthenol und Propylenglycol | 3,00 % |
| Melanosponges P 031 A | 0,20 % |
| Aloe vera Gel | 0,10 % |
| Dinatrium-EDTA | 0,05 % |
| PEG-8 | 5,00 % |
| Carbomer | 0,20 % |
| Polymethylsilsesquioxane (Tospearl 145) | 1,00 % |
| Phenonip | 0,50 % |
| Euxyl K 400 | 0,50 % |
| Parfümöl | 0,50 % |
| Deoinisiertes Wasser | ad 100 |

Die Herstellung der Formulierung erfolgte durch Emulgierung einer wäßrigen und einer öligen Phase bei üblichen Temperaturen zwischen 50 und 75 °C durch Homogenisierung. Dabei wurden in die wäßrige Phase Dinatrium-EDTA, PEG 8 und Aloe vera sowie das Polymethylsilsesquioxan aufgenommen und danach das Carbomer. Nach Erhalt einer Dispersion und Erwärmung auf etwa 70 °C wurde die 2-Phenylbenzimidazol-5-sulfonsäure hinzugegeben.

Die ölige Phase wurde durch Vermischen von Phenyltrimethicon, Tocopherylacetat, Octyldodecylneopentanoat, Bisabolol, Isostearylneopentanoat und BHT hergestellt und auf 60 °C vorerwärmt. Gleichzeitig wurde Octylmethoxycinnamat und Benzophenon bei leichter Erwärmung vermischt und zu der öligen Phase hinzugegeben. Schließlich wurde noch Pemulen TR 2 unter guter Homogenisierung zugesetzt bis zum Erhalt einer Dispersion der öligen Phase.

Nach Emulgierung von öliger und wäßriger Phase und Abkühlung auf etwa 40 °C wurden Melanosponges P031A und Panthenol/Propylenglycol zugesetzt, gefolgt von den Schutzmitteln und den Parfümkomponenten.

### Beispiel 2 Liposome

Ein Phospholipid mit löslichem tierischem Kollagen und hydrolysierten Mucopolysacchariden wurde in eine wäßrige Suspension von Polymethylsilsesquioxan und Octyldodecylneopentanoat eingetragen. Nach guter Homogenisierung wurde unter Rühren die erhaltene Suspension zu der gemäß Beispiel 1 erhaltenen Dispersion mit den restlichen Bestandteilen hinzugegeben und weiter homogenisiert, so daß sich insgesamt ein Anteil der Liposome von etwa 5 % ergab.

### Beispiel 3 Aggregate

Ein Perfluorcarbon (Perfluordekalin), vermischt mit Glycerin und Propylenglycol, wurde mit Polymethylsilsesquioxan und Octyldodecylneopentanoat vermischt, und dazu wurde ein Phospholipid mit einem Phosphatidylcholingehalt von 40 % gegeben. Das Gemisch wurde gut homogenisiert. Die dabei gebildeten asymmetrischen lamellaren Aggregate wurden der Dispersion mit den restlichen Bestandteilen der Formulierung gemäß Beispiel 1 hinzugegeben und weiter homogenisiert, so daß sich insgesamt ein Anteil der Liposome von etwa 5 % ergab.

### Vergleichsbeispiel 1

Es wurde wie im Beispiel 1 verfahren, mit Ausnahme dessen, daß das feinteilige vernetzte Poly(Methylsiloxan) (CTFA-Name: Polymethylsilsesquioxane) nicht enthalten war, und der Anteil an Octyldodecylneopentanoat nur 2,00 % betrug. Das Vermischen wurde in ähnlicher Weise wie im Beispiel 1 durchgeführt. Dabei erhielt man für die Formulierung des Vergleichsbeispiels einen Lichtschutzfaktor (vor dem Eintauchen) von kleiner als 16,16 und nach dem Eintauchen von kleiner als 15,84.

Bei der Formulierung gemäß Beispiel 1 erhielt man einen Lichtschutzfaktor (vor dem Eintauchen) von 19,80 und einen Lichtschutzfaktor nach dem Eintauchen von 17,00.

Die Messung des Lichtschutzfaktors erfolgte nach der von der US-Gesundheitsbehörde (FDA) vorgeschriebenen Vorschrift bei der unter Bestrahlung mit dem Solar-Ultraviolettsimulator, Modell 10 S mit einer 150 W Xenon-Bogenlampe eine Gruppe von 5 Personen mit der erfindungsgemäßen Sonnenschutzzubereitung sowie der des Vergleichsbeispiels untersucht worden war.

Die Ergebnisse zeigen, daß durch Zusatz von Poly(Methylsiloxan) eine deutliche Erhöhung des Lichtschutzfaktors erreichbar war.

## Patentansprüche

1. Sonnenschutzzubereitung mit erhöhtem Lichtschutzfaktor, bestehend aus
(A) einem feinteiligen, vernetzten Poly(methylsiloxan) mit einer Teilchengröße im Bereich von 1 bis 10 µm, wobei das Poly(Methylsiloxan) folgende Parameter aufweist:
- Gewichtsverlust bei 350 °C von 3 % oder weniger und bei 900 °C von 12 % oder weniger
- keine Veränderung des Teilchendurchmessers bei Calcinierung in Luft über vier Stunden bis etwa 350 °C, Verringerung des Teilchendurchmessers bis 900 °C um etwa 25% oder weniger, jeweils unter Beibehaltung des im wesentlichen runden Aufbaus
- Fehlen eines endothermen Peaks beim Aufheizen in Luft mit einer Heizgeschwindigkeit von 10 °C/Minute
- Beständigkeit gegen organische Lösungsmittel auf Basis von Alkoholen, Ketonen, Estern, Aromaten und chlorierten Kohlenwasserstoffen;
(B) Octyldodecylneopentanoat;
(C) Formulierungshilfen; und
(D) Additiven;
neben üblichen Lichtschutzmitteln;
wobei das Verhältnis von (A) zu (B) im Bereich von 1 : 2 bis 10 liegt, und der Anteil von (A) und (B) im Bereich von 3 bis 12 Gewichts-% liegt, bezogen auf die Gesamtzusammensetzung.

2. Sonnenschutzzubereitung nach Anspruch 1, dadurch gekennzeichnet, daß die mittlere Teilchengröße 4,5 µm beträgt.

3. Sonnenschutzzubereitung nach Anspruch 1, dadurch gekennzeichnet, daß das Verhältnis (A) zu (B) im Bereich von 1 : 2,5 bis 8, vorzugsweise im Bereich von 1 : 5 liegt.

4. Sonnenschutzzubereitung nach Anspruch 1, dadurch gekennzeichnet, daß der Anteil von (A) und (B) an der Gesamtzusammensetzung im Bereich von 4 bis 8 Gewichts-% liegt.

5. Sonnenschutzzubereitung nach Anspruch 1, dadurch gekennzeichnet, daß das feinteilige vernetzte Poly(Methylsiloxan) zusammen mit der Komponente (B) verkapselt in Liposomen vorliegt.

6. Sonnenschutzzubereitung nach Anspruch 1, dadurch gekennzeichnet, daß (A) und (B) in asymmetrisch lamellaren Aggregaten vorliegen, die aus Phospholipiden und mit Sauerstoff beladenen Fluorcarbonen oder Fluorcarbongemischen bestehen, wobei der Anteil von Fluorcarbonen im Bereich von 0,2 bis 100% w/v liegt und die einen Phosphatidylcholingehalt der Lipidfraktion von 30 bis 99 Gewichts-% haben, mit einer Hautpenetrierung der Aggregate in Abhängigkeit von der kritischen Löslichkeitstemperatur der Fluorcarbone.

7. Sonnenschutzzubereitung nach Anspruch 1, dadurch gekennzeichnet, daß die Formulierung als Lotion oder als Spray vorliegt.

## Claims

1. A sun protection preparation with an increased sun protection factor consisting of
(A) a fine-particle, crosslinked poly(methylsiloxane) with a particle size ranging from 1 to 10 µm, and comprising the following parameters:
- its weight loss is 3% or less at 350 °C, and 12% or less at 900 °C;
- the particle diameter does not change when calcined in air for four hours up to about 350 °C and is reduced by 25% or less at 900 °C while retaining its generally circular structure;
- there is no endothermic peak when heated in air at a heating rate of 10 °C per minute;
- it is resistant to organic solvents based on alcohols, ketones, esters, aromates, and chlorinated hydrocarbons.
(B) octyldodecyl neopentanoate;
(C) formulation aids; and
(D) additives;
besides common sun protection agents;
wherein the ratio of (A) to (B) ranges from 1 : 2 to 10, and the portion of (A) and (B) ranges from 3 to 12 wt %, relative to the total composition.

2. The sun protection preparation according to claim 1 wherein the mean particle size is 4.5 µm.

3. The sun protection preparation according to claim 1 wherein the ratio of (A) to (B) ranges from 1 : 2.5 to 8, and preferably is 1 : 5.

4. The sun protection preparation according to claim 1 wherein the portion of (A) and (B) ranges from 4 to 8 wt %, relative to the total composition.

5. The sun protection preparation according to claim 1 wherein the fine-particle, crosslinked poly(methylsiloxane) and component (B) are encapsulated in liposomes.

6. The sun protection preparation according to claim 1 wherein (A) and (B) are present in the form of asymmetric lamellar aggregates consisting of phospholipids and fluorocarbons or fluorocarbon mixtures charged with oxygen, the portion of fluorocarbons ranging from 0.2 to 100% w/v, and the phosphatidylcholine content of the lipid fraction being 30 to 99 wt %, and wherein the penetration of the skin by said aggregates is dependent upon the critical solubility temperature of the fluorocarbons.

7. The sun protection preparation according to claim 1 wherein the formulation is present in the form of a lotion or spray.

## Revendications

1. Composition de protection solaire à indice de protection solaire renforcé, se composant de:
(A) un poly(méthylsiloxane) réticulé, en fines particules, d'une grosseur de particules de l'ordre de 1 à 10 µm, ledit poly(méthylsiloxane) présentant les paramètres suivants:
- perte de poids de 3 % ou moins à 350 °C et de 12 % ou moins à 900 °C;
- pas de modification du diamètre des particules en cas de calcination par de l'air sur quatre heures jusqu'à environ 350 °C, diminution du diamètre des particules de 25 % ou moins, environ, jusqu'à 900 °C, toujours avec maintien de la structure sensiblement ronde;
- absence de pic endothermique lors du chauffage dans de l'air, avec une vitesse de chauffage de 10 °C/minute;
- résistance aux solvants organiques à base d'alcools, cétones, esters, séries aromatiques et hydrocarbures chlorés.
(B) octyldodécylnéopentanoate;
(C) aides de formulation; et
(D) additifs;
le rapport de (A) à (B) étant de l'ordre de 1: 2 à 10, et la proportion de (A) et (B) de l'ordre de 3 à 12 % en poids, par rapport à la composition globale.

2. Composition de protection solaire selon la revendication 1, caractérisée en ce que la grosseur moyenne des particules est de 4,5 µm.

3. Composition de protection solaire selon la revendication 1, caractérisée en ce que le rapport de (A) à (B) se situe dans l'intervalle de 1 : 2,5 à 8, de préférence dans l'intervalle de 1 : 5.

4. Composition de protection solaire selon la revendication 1, caractérisée en ce que la part de (A) et de (B) dans la composition globale se situe dans l'intervalle de 4 à 8 % en poids.

5. Composition de protection solaire selon la revendication 1, caractérisée en ce que le poly(méthylsiloxane) réticulé en fines particules est enrobé avec le composant (B) dans des liposomes.

6. Composition de protection solaire selon la revendication 1, caractérisée en ce que (A) et (B) se présentent sous la forme d'agrégats lamellaires asymétriques qui se composent de phospholipides et de fluorocarbones ou mélanges de fluorocarbones chargés en oxygène, la part de fluorocarbones se situant dans l'intervalle de 0,2 à 100 % w/v, et qui présentent une teneur en phosphatidylcholine de la fraction de lipides de 30 à 99 % en poids, avec une pénétration des agrégats dans la peau dépendant de la vitesse de solubilité critique des fluorocarbones.

7. Composition de protection solaire selon la revendication 1, caractérisée en ce que la formulation se présente sous forme de lotion ou de spray.
